# EUROPÄISCHE PATENTSCHRIFT

(11) **EP 2 374 867 B1**
(45) Veröffentlichungstag und Bekanntmachung des Hinweises auf die Patenterteilung: **16.03.2016**
(21) Anmeldenummer: 11005576.1
(22) Anmeldetag: 11.05.2009
(51) Int. Cl.: C12M 1/107, C12M 1/04

(54) **Verfahren und Vorrichtung zum Betreiben einer Fermentationsanlage**
Method and device for operating a fermentation assembly
Procédé et dispositif destinés au fonctionnement d'une installation de fermentation

(43) Veröffentlichungstag der Anmeldung: 12.10.2011
(62) Teilanmeldung aus: 09006326.4
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A- 1 428 868
- EP-A- 1 736 535
- EP-A- 1 811 015
- EP-A- 1 997 875
- WO-A-02/06439
- WO-A-2005/054423

## Beschreibung

Die Erfindung betrifft ein Verfahren zum Betreiben einer Fermentationsanlage nach dem Oberbegriff des Anspruchs 1.

Bei entsprechenden Verfahren wird ein Substrat in einem Fermenter mit Perkolat durchströmt, welches zumeist am Boden des Fermenters aufgefangen, in einen Perkolatbehälter zurückgeführt und von dort aus dem Fermenter wieder zugeführt werden kann. Hierdurch entsteht im Fermenter und auch im Perkolatbehälter methanhaltiges Biogas, welches aus dem Fermenter und/oder dem Perkolatbehälter abgeführt und einer Biogasaufbereitung zur Erhöhung der Methankonzentration zugeführt wird. Bei der Biogasaufbereitung wird das zu gewinnende Biogas von weiteren unerwünschten Bestandteilen getrennt und der weiteren Verwendung zugeführt. Bei der Aufbereitung entsteht unter anderem eine erhebliche Menge an CO₂.

Um das Biogas aus dem Fermenter am Ende des Prozesses der Biogasaufbereitung zuzuführen, muss das Biogashaltige Volumen oberhalb des Substrates nach dem Fermentationsprozess mit einem Spülgas gespült werden, um das Biogas aus dem Volumen abzufahren. Aus EP 1 997 875 A1 ist bekannt, hierzu aus der Aufbereitung stammendes CO₂-haltiges Gas einzusetzen.

Während des Fermentationsprozesses entstehen auch nicht unerhebliche Mengen an Biogas, welche allerdings im Substrat "gefangen" sind. Diese werden beim Materialhandling oder der Gärrestnachbearbeitung freigesetzt. Die freigesetzten Biogasanteile, insbesondere Methan, gelangen so unkontrolliert in die Atmosphäre.

Die EP 1 428 868 A1 (D1) offenbart eine Fermentationsanlage, bei der es möglich ist, Fermenter mit einem Biogas, welches in Fermentern der dortigen Fermentationsanlage produziert worden ist, zu spülen. Bei dem Biogas handelt es sich jedoch um hoch methanhaltiges, in einer Verwertungseinrichtung verwertbares, also insbesondere brennbares, Biogas, welches ebenfalls hoch methanhaltiges Biogas aus dem zu spülenden Fermenter verdrängt. Durch eine derartige Spülung wird daher auch keine erhöhte Methanausbeute erzielt. Das Spülverfahren dient im Fall der D1 (EP 1 428 868 A1) lediglich dazu, den Wärmeeintrag in den Fermenter kostengünstig zu gestalten.

Die EP 1 811 015 A1 offenbart eine Flüssigfermentationsanlage, bei der das in einem Fermenter produzierte Biogas im Rahmen einer Kreislaufführung im gleichen Fermenter als Spülgas genutzt wird. Auch in diesem Fall wird Biogas durch Biogas mit vergleichbar hoher Methankonzentration verdrängt, die Methanausbeute somit durch die Spülung nicht erhöht. Die Biogasspülung sorgt hier lediglich für ein besseres Durchmischen der flüssigen Biomasse und übernimmt so prinzipiell die Funktion eines Rührwerks.

Darüber hinaus offenbaren die Druckschriften EP 1 736 535 A, WO 02/06439 A und WO 2005/054423 A Fermentationsanlagen. Allen dort offenbarten Fermentationsanlagen und Verfahren ist es jedoch gemein, dass die Problematik des im Substrat gefangenen Methans weiterhin besteht.

Der Erfindung liegt daher die Aufgabe zugrunde, ein Verfahren anzugeben, bei dem die erwähnten Nachteile nicht auftreten und der Methanschlupf auf ein Minimum reduziert werden kann.

Erfindungsgemäß wird diese Aufgabe mit den Merkmalen des Anspruchs 1 gelöst. Vorteilhafte Ausführungsformen finden sich in den Unteransprüchen.

Beim erfindungsgemäßen Verfahren wird das CO₂-haltige Spülgas durch das Substrat geleitet. Bevorzugt wird unter hohem Druck das Spülgas im Bodenbereich möglichst flächig in den Fermenter eingeleitet. So wird das Substrat durchspült und darin vorhandene Biogasanteile gelangen insbesondere in das Volumen oberhalb des Substrates und können in bekannter Weise zur Biogasaufbereitung abgefahren werden. Das CO₂-haltige Spülgas kann dabei als Abgas aus einer Biogasaufbereitung entnommen werden. Alternativ oder ergänzend hierzu ist es möglich, in einem anderen Fermenter durch den Fermentationsprozess entstandenes CO₂-haltiges Biogas aus einer frühen Phase des Fermentationsprozesses in den zu spülenden Fermenter einzuleiten.

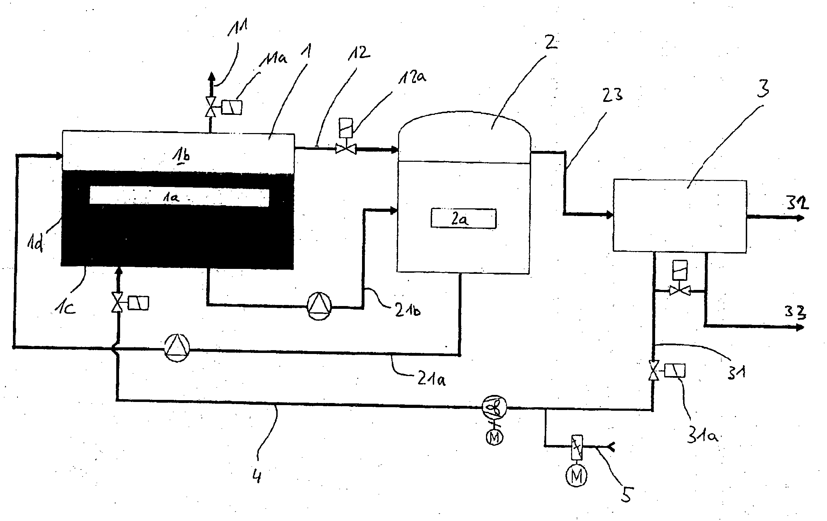

Eine Verfahrensvariante außerhalb des Schutzumfangs von Anspruch 1 wird nachfolgend anhand des in der Figur gezeigten Ausführungsbeispiels schematisch näher erläutert.

Ein Fermenter 1 wird mit einem Substrat 1a beschickt. Gezeigt ist lediglich ein Fermenter, jedoch sind Anlagen mit einer Mehrzahl Fermenter möglich. Der Fermenter 1 ist mit einem Perkolatbehälter 2 verbunden durch eine Zuführungsleitung 21a, mit der Perkolat 2a in den Fermenter 1 eingebracht wird und während des Fermentationsprozesses durch das Substrat 1a sickert. Das Perkolat wird im unteren Bereich des Fermenters 1 aufgefangen und über eine Rücklaufleitung 21b wieder dem Perkolatbehälter 2 zugeführt.

Beim Fermentationsprozess entsteht Biogas, welches sich im Fermenter 1 vorrangig im Volumen 1b über dem Substrat 1a sammelt. Zudem befindet sich auch Biogas im Substrat 1a, welches darin eingeschlossen ist und nicht ohne weiteres in das Volumen 1b gelangen kann. Des Weiteren entsteht auch im Perkolatbehälter 2 zusätzliches Biogas. Das im Fermenter in den Bereichen 1b und 1a vorhandene Biogas wird durch den erfindungsgemäßen Spülvorgang abgefahren. Hierzu ist zum einen eine Biogasleitung 12 zwischen Fermenter 1 und Perkolatbehälter 2 und eine weitere Leitung 23 zwischen dem Perkolatbehälter 2 und einer Aufbereitungseinrichtung 3 vorgesehen. Zum Spülen wird das in der Aufbereitungseinrichtung 3 bei der Aufbereitung entstehende CO₂-haltige Gas verwendet. Im Folgenden wird unter CO₂-haltigem Gas ein Gas verstanden, welches überwiegend CO₂ enthält. Es kann auch ausschließlich CO₂ enthalten, andere Bestandteile sind aber auch denkbar.

Bei der Biogasaufbereitung entsteht neben hochkonzentriertem Methan, welches über die Leitung 32 zur Verfügung gestellt wird, überwiegend CO₂-haltiges Abgas, das über die Leitung 33 abgeführt werden kann, oder über die Leitung 31 in die zum Fermenter 1 führende Spülleitung 4 eingeleitet wird. Der Spülanschluss am Fermenter 1 ist so positioniert, dass das durch die Spülleitung 4 zugeführte Spülgas durch das Substrat hindurch geleitet wird. Bevorzugt ist hierzu im Boden lc oder Bodenbereich des Fermenters 1 oder an der Seite in einem noch von Substrat 1a bedeckten Bereich 1d des Fermenters 1 der Anschluss für die Spülleitung 4 vorgesehen. Zudem kann eine Verteileinrichtung (nicht gezeigt) vorgesehen sein, um das Spülgas an mehreren Stellen durch das Substrat zu leiten. Dies kann im Fermenter 1 oder bereits durch Aufteilen der Spülleitung 4 vor dem Fermenter 1 geschehen. In jedem Fall kann eine Mehrzahl von Spülanschlüssen vorgesehen werden.

Das CO₂-haltige Spülgas wird unter Druck in den Fermenter 1 eingeleitet, sodass noch im Substrat 1a festgehaltenes Biogas aus dem Substrat 1a verdrängt wird. Dabei wird der Anschluss zur Biogasleitung 12 bzw. ein entsprechendes Ventil 12a geöffnet, sodass das aus dem Substrat 1a und dem substratfreien Volumen 1b verdrängte Biogas über den Perkölatbehälter 2 und die Leitung 23 zur Aufbereitungseinrichtung 2 abgefahren wird. Ist das Biogas aus dem Fermenter 1 abgefahren bzw. ist ein vorgegebener Umschaltpunkt erreicht, der sich beispielsweise aus einem Schwellenwert der Biogas(Methan)-Konzentration ergeben kann, wird das Ventil 12a in der Leitung 12 wieder geschlossen und das Abluftventil 11a zur Abluftleitung 11 geöffnet; das Ventil 31a der CO₂-Leitung wird geschlossen und die CO₂-Zufuhr wird gestoppt. Es erfolgt nun weitere Spülung durch Frischluft, welche aus einer Frischluftleitung 5 der Spülleitung 4 zugeführt wird. Die durch die Abluftleitung 11 aus dem Fermenter entweichende Abluft wird dann einer hier nicht näher beschriebenen Abluftbehandlung und/oder -ableitung unterzogen.

Bei der gezeigten Verfahrensvariante wird aus einem weiteren Fermenter dort im Fermentationsprozess entstandenes CO₂-haltiges Biogas, welches während einer frühen Phase des Fermentionsprozesses angefallen ist als Spülgas verwendet. Erfindungsgemäß wird Biogas verwendet welches in einem anderen Fermenter (nicht gezeigt) entsteht. In der ersten Phase des Fermentationsprozesses entsteht eine große Menge an CO₂ im Biogas. Dieses CO₂-haltige Biogas aus einem Fermenter kann zum Spülen eines anderen Fermenters eingesetzt werden, indem etwa der Spülgasanschluss des zu spülenden Fermenters mit der Biogasleitung eines anderen Fermenters verbunden wird.

Mit dem erfindungsgemäßen Verfahren kann der Methanschlupf durch das erfindungsgemäße Verfahren deutlich reduziert werden. Zudem kann die Effizienz der Biogasgewinnung gesteigert werden, weil auch das im Substrat noch vorhandene Biogas der Aufbereitung zugeführt werden kann.

## Patentansprüche

1. Verfahren zum Betreiben einer Fermentationsanlage mit wenigstens einem zu spülenden Fermenter (1), der mit einem Substrat (1a) beschickt ist, wobei das Substrat (1a) mit Perkolat (2a) aus einem Perkolatbehälter (2) perkoliert wird und über den Perkolationsprozess im zu spülenden Fermenter (1) oder/und im Perkolatbehälter (2) entstehendes Biogas abgezogen wird, wobei aus einem weiteren Fermenter (1) dort im Fermentationsprozess entstandenes CO₂-haltiges Biogas, welches während einer frühen Phase des Fermentationsprozesses angefallen ist und daher einen erhöhten CO₂-Gehalt aufweist, mittels einer Spülleitung (4) zum Abfahren des im zu spülenden Fermenter (1) entstandenen Biogases in den zu spülenden Fermenter (1) eingeleitet wird, wobei das CO₂-haltige Spülgas so in den zu spülenden Fermenter (1) eingeleitet wird, dass es durch das Substrat (1a) hindurch geleitet wird, indem ein Spülgasanschluss des zu spülenden Fermenters mit einer Biogasleitung des weiteren Fermenters verbunden wird.

2. Verfahren nach Anspruch 1,
**dadurch gekennzeichnet,**
**dass** das CO₂-haltige Spülgas so durch das Substrat geleitet wird, dass im Substrat (1a) vorhandenes Gas in das substratfreie Volumen des Fermenters (1) verdrängt wird.

3. Verfahren nach einem der vorherigen Ansprüche,
**dadurch gekennzeichnet,**
**dass** das CO₂-haltige Spülgas in den Boden des Fermenters (1) eingeleitet wird.

4. Verfahren nach Anspruch 3,
**dadurch gekennzeichnet,**
**dass** das CO₂-haltige Spülgas im Bereich des Bodens des Fermenters (1) verteilt wird, sodass das Substrat (1a) im Wesentlichen gleichmäßig von unten nach oben mit dem CO₂-haltigen Spülgas durchströmt wird.

5. Verfahren nach einem der vorherigen Ansprüche,
**dadurch gekennzeichnet,**
**dass** nach dem Spülen mit CO₂-haltigem Spülgas Frischluft über die Spülleitung (4) in den Fermenter (1) eingeleitet wird.

6. Verfahren nach einem der vorherigen Ansprüche,
**dadurch gekennzeichnet,**
**dass** während des Einleitens des CO₂-haltigen Spülgases der Biogasabzug über eine Abzugsleitung (12, 23) erfolgt.

7. Verfahren nach einem der vorherigen Ansprüche,
**dadurch gekennzeichnet,**
**dass** nach Beendigen des Spülvorgangs mit CO₂-haltigem Gas der Biogasabzug gesperrt und eine Abluftleitung (11) geöffnet wird.

## Claims

1. Method for operating a fermentation assembly having at least one fermenter (1) which is loaded with a substrate (1a) and which is to be flushed, wherein the substrate (1a) is percolated with a percolate (2a) from a percolate container (2), and biogas forming in the fermenter (1) which is to be flushed, and/or in the percolate container (2) is extracted through the percolation process, wherein CO₂-containing biogas arising in a further fermenter (1) in the fermentation process and which has accumulated during an early phase of the fermentation process and therefore has an increased C₂ content, is introduced by means of a flushing pipeline (4) for discharging the biogas, which has arisen in the fermenter (1) which is to be flushed, into the fermenter (1) which is to be flushed, wherein the CO₂- containing flushing gas is introduced into the fermenter (1) which is to be flushed, so that it is directed through the substrate (1a), in that a flushing gas connection of the fermenter, which is to be flushed, is connected to a biogas line of the further fermenter.

2. Method according to claim 1
**characterised in that**
the CO₂-containing flushing gas is directed through the substrate so that gas present in the substrate (1a) is forced into the substrate-free volume of the fermenter (1).

3. Method according to one of the preceding claims,
**characterised in that**
the CO₂-containing flushing gas is introduced into the bottom of the fermenter (1).

4. Method according to claim 3
**characterised in that**
the CO₂-containing flushing gas is dispersed in the region of the bottom of the fermenter (1) so that the CO₂-containing flushing gas flows substantially uniformly up through the substrate (1a) from bottom to top.

5. Method according to one of the preceding claims
**characterised in that**
after flushing with CO₂-containing flushing gas, fresh air is introduced into the fermenter (1) via the flushing pipeline (4).

6. Method according to one of the preceding claims
**characterised in that**
during the introduction of the CO₂-containing flushing gas the biogas is extracted via an extraction pipeline (12, 23).

7. Method according to one of the preceding claims
**characterised in that**
at the end of the flushing process with CO₂-containing gas the biogas extraction is blocked and an exhaust pipeline (11) is opened.

## Revendications

1. Procédé de fonctionnement d'une installation de fermentation avec au moins un fermenteur à rincer (1), qui est chargé avec un substrat (!a), sachant que le substrat (1a) est percolé avec un percolat (2a) en provenance d'un conteneur de percolat (2) et que le biogaz généré dans le fermenteur à rincer (1) ou / et dans le conteneur de percolat (2) est extrait selon un processus de percolation, sachant que, à partir d'un autre fermenteur (1), du biogaz contenant du CO₂, y généré au cours du processus de fermentation, au cours d'une phase antérieure du processus de fermentation et présentant de ce fait une teneur en CO-2 surélevée, est introduit dans le fermenteur à rincer (1), par une conduite de rinçage (4) destinée à conduire le biogaz généré dans le fermenteur à rincer (1), sachant que le gaz de rinçage contenant du CO₂ est introduit dans le fermenteur à rincer (1) de sorte qu'il passe à travers le substrat (1a), un raccord de gaz de rinçage du fermenteur à rincer étant relié à une conduite de biogaz de l'autre fermenteur.

2. Procédé selon la revendication 1,
**caractérisé en ce que**
le gaz de rinçage contenant du CO₂ est conduit à travers le substrat (1a) de telle manière que le gaz, présent dans ledit substrat, soit refoulé dans le volume exempt de substrat du fermenteur (1).

3. Procédé selon l'une des revendications précédentes,
**caractérisé en ce que**
le gaz de rinçage contenant du CO₂ est introduit dans le fond du fermenteur (1).

4. Procédé selon la revendication 3,
**caractérisé en ce que**
le gaz de rinçage contenant du CO₂ est réparti dans la région du fond du fermenteur (1) de telle manière que le substrat (1a) soit traversé sensiblement régulièrement, du bas vers le haut, par le gaz de rinçage contenant du CO₂.

5. Procédé selon l'une des revendications précédentes,
**caractérisé en ce que**,
après le rinçage avec le gaz de rinçage contenant du CO₂, de l'air frais est introduit dans le fermenteur (1) par l'intermédiaire de la conduite de rinçage (4).

6. Procédé selon l'une des revendications précédentes,
**caractérisé en ce que**,
pendant l'introduction du gaz de rinçage contenant du CO₂, l'extraction du biogaz s'effectue par l'intermédiaire d'une conduite d'extraction (12, 23).

7. Procédé selon l'une des revendications précédentes,
**caractérisé en ce que**,
après la fin de l'opération de rinçage avec le gaz de rinçage contenant du CO₂, la sortie du biogaz est fermée et une conduite d'air d'échappement (11) est ouverte.
